**Europäisches Patentamt**

**European Patent Office**

**Office européen des brevets**

(11) Numéro de publication: **0 108 675**
**B1**

# (12) FASCICULE DE BREVET EUROPEEN

(45) Date de publication du fascicule du brevet:
**03.07.85**

(21) Numéro de dépôt: **83402072.9**

(22) Date de dépôt: **25.10.83**

(51) Int. Cl.⁴: **C 07 C 51/60,** C 07 C 51/58,
C 07 C 53/48

(54) **Procédé de préparation de chlorure de trifluoroacétyle.**

(30) Priorité: **05.11.82 FR 8218567**

(43) Date de publication de la demande:
**16.05.84 Bulletin 84/20**

(45) Mention de la délivrance du brevet:
**03.07.85 Bulletin 85/27**

(84) Etats contractants désignés:
**CH DE FR GB IT LI NL**

(56) Documents cités:
**FR - A - 1 228 408**
**GB - A - 515 963**

(73) Titulaire: **ATOCHEM, 12/16, allée des Vosges,
F-92400 Courbevoie (FR)**

(72) Inventeur: **Cheminal, Bernard, Beauséjour
St.-Irénée 26, rue Soeur Bouvier, F-69005 Lyon (FR)**
Inventeur: **Mathais, Henri Hameau de St. Didier - Villa
No 2, Chemin de l'Indiennerie,
F-69370 Saint-Didier-au-Mont-d'Or (FR)**
Inventeur: **Thomarat, Marc, 22 Boulevard de l'Europe,
F-69310 Pierre Bénite (FR)**

(74) Mandataire: **Leboulenger, Jean et al, Société ATOCHEM
Service Propriété Industrielle Tour Manhattan,
F-92091 Paris La Défense 2 Cédex 22 (FR)**

ACTORUM AG

## Description

L'invention concerne la préparation de chlorure de trifluoroacétyle par chloration catalytique de trifluoroacétaldéhyde (fluoral) par le chlore. L'invention concerne plus particulièrement la préparatin de chlorure de trifluoroacétyle par chloration du fluoral par le chlore en phase gazeuse sur un catalyseur charbon actif.

Le chlorure de trifluoroacétyle est un produit industriel intéressant puisqu'il permet par exemple d'obtenir, par hydrolyse selon des techniques connues, l'acide trifluoroacétique employé en pharmacie, comme catalyseur de dimérisation des hydrocarbures aliphatiques insaturés ou comme principe actif dans les formulations pesticides.

Il est connu que l'on peut synthétiser le chlorure de trifluoroacétyle par des procédés non catalytiques.

C'est ainsi que le brevet français No 2226380 décrit l'oxydation en phase gazeuse du dichloro-1,1 trifluoroéthane en chlorure de trifluoroacétyle, par l'oxygène sous irradiation ultraviolette. Un tel procédé exige des mesures de sécurité contraignantes pour éviter le danger d'explosion, une grande complexité de l'appareillage photochimique et une importante consommation d'énergie sous forme de lumière et de frigories pour n'aboutir qu'à une faible productivité.

Le brevet français No 1385111 concerne un procédé de chloration photochimique du fluoral liquide pur vers −30°C en présence de lumière ultraviolette. Une telle méthode ne peut être aisément mise en œuvre industriellement par suite de la rapidité de polymérisation du fluoral, qui rend son stockage difficile et qui exige, ce que ne permet pas de réaliser le brevet français, qu'il soit rapidement transformé avec un rendement quantique élevé.

Il est connu aussi de synthétiser le chlorure de trifluoroacétyle, par des voies catalytiques à partir de composés autres que le fluoral.

Le brevet français No 2038257 décrit l'oxydation catalytique du trichloro-1,1,1 trifluoroéthane par l'anhydride sulfurique. Un tel procédé présente le double inconvénient de mettre en œuvre des catalyseurs toxiques à base de sulfate de mercure, et de conduire à la formation d'un sous-produit gênant: le chlorure de sulfuryle.

Le brevet français No 2169221 concerne un procédé de préparation d'acide trifluoroacétique dont une étape consiste à transformer catalytiquement le fluorure de trifluoroacétyle en chlorure de trifluoroacétyle en présence de tétrachlorure de carbone, de chloroforme ou de trichlorofluorométhane. Un tel mode d'obtention du chlorure de trifluoroacétyle présente l'inconvénient majeur de conduire inévitablement à la formation de coproduits, comme, en particulier, le trichlorofluorométhane dans le cas de l'emploi de tétrachlorure de carbone.

Le procédé selon l'invention permet de remédier aux inconvénients des techniques connues et consiste à préparer le chlorure de trifluoroacétyle par chloration du fluoral par le chlore en phase gazeuse à l'aide d'un catalyseur charbon actif.

Un tel procédé selon l'invention permet d'atteindre une conversion totale du fluoral et des rendements élevés en chlorure de trifluoroacétyle.

Le fluoral peut être employé pur ou sous sa forme de composé d'addition avec l'acide chlorhydrique (chlorhydrate). Sous l'une ou l'autre forme, le fluoral gazeux peut être dilué par de l'acide chlorhydrique et/ou un inerte tel que l'azote ou le chlorotrifluorométhane.

Le rapport molaire acide chlorhydrique/fluoral peut être de 0 à 5. Le rapport molaire diluant inerte/fluoral peut être de 0 à 5. Lorsque l'acide chlorhydrique et le diluant inerte sont présents simultanément la somme des rapports molaires acide chlorhydrique/fluoral et diluant inerte/fluoral n'est pas supérieure à 5. A titre d'exemple, le rapport diluant inerte/fluoral sera de préférence de 3 à 4 lorsque le rapport molaire acide chlorhydrique/fluoral sera de 1. L'azote, le chlorotrifluorométhane peuvent être choisis, par exemple, comme diluant inerte.

Le rapport molaire chlore/fluoral est généralement de 1 à 2.

La température de réaction est le plus souvent comprise entre 130 et 250°C.

La réaction est effectuée à une pression pouvant différer de la pression atmosphérique mais préférentiellement comprise entre 1 et 5 bars absolus.

La réaction peut être effectuée en lit fixe ou en lit fluidisé. Lorsqu'elle est effectuée en lit fixe, le temps de contact, exprimé comme étant le temps en secondes nécessaire pour introduire dans le réacteur un volume de mélange de réactifs mesuré dans les conditions normales de température et de pression, égal au volume apparent du catalyseur, est généralement compris entre 5 et 30 s.

Lorsque la réaction est effectuée en lit fluidisé, la vitesse des gaz constitués par le mélange des réactifs entrant dans la zone de réaction est telle qu'elle corresponde à un débit le plus souvent égal à 1,5 à 3 fois le débit minimum de fluidisation du catalyseur. Le catalyseur est choisi parmi la gamme connue des charbons actifs.

Les exemples suivants, donnés à titre non limitatif, illustrent le procédé selon l'invention.

Pour chacun des essais décrits, le mélange gazeux sortant du réacteur est analysé par chromatographie gazeuse sur une phase silicone après neutralisation de l'acide chlorhydrique sur du phosphate disodique.

*Exemple 1:*

300 ml de catalyseur constitué de charbon actif de la Société Norit (surface spécifique 947 m²/g, volume poreux: 0,59 cm³/g) en grains de 0,8 mm, sont placés dans un réacteur comprenant essentiellement un tube en Inconel de 40 mm ∅ et de 240 mm de hauteur utile, et pourvu d'un moyen de chauffage.

Après un séchage à 300°C sous courant d'azote puis un passage de 0,577 mol/h de chlore durant 1 h, un mélange gazeux contenant, pour 1 mol de fluoral, 1,28 mol de chlore et 4,93 mol d'acide chlorhydrique est introduit dans le réacteur fonctionnant à pression atmosphérique et à 185°C, à raison de 48,5 Nl/h.

Le taux de conversion du fluoral est de 51% et le rendement molaire de chlorure de trifluoroacétyle, par rapport au fluoral transformé, est de 85,3%.

*Exemple 2:*

En opérant dans le même appareillage et avec le même catalyseur que pour l'exemple 1, mais en ne mettant en œuvre que 100 ml de catalyseur à la température de 136°C et en introduisant dans le réacteur 10 Nl/h d'un mélange de réactifs renfermant, pour 1 mol de fluoral, 1,4 mol de chlore et 1,22 mol d'acide chlorhydrique, le taux de transformation du fluoral est de 80% et le rendement de chlorure de trifluoroacétyle de 60%.

*Exemple 3:*

300 ml de catalyseur de l'exemple 1 présenté sous forme de grains de 125 à 210 μm sont placés dans l'appareillage décrit dans l'exemple 1 muni à sa base d'un système de répartition de flux gazeux assurant la qualité de la fluidisation du catalyseur. Un mélange gazeux contenant, pour 1 mol de fluoral, 1,3 mol de chlore, 1 mol d'acide chlorhydrique et 3 mol d'azote est introduit dans le réacteur à raison de 32,8 Nl/h.

La température à laquelle s'effectue la réaction est de 190°C. Le taux de conversion du fluoral est de 98,6% et le rendement de chlorure de trifluoroacétyle est de 92,1%.

*Exemple 4:*

Dans les mêmes conditions que dans l'exemple 3, mais en opérant avec des rapports molaires chlore/fluoral et azote/fluoral de 1,90 et 3,65 respectivement, la conversion du fluoral est totale et le rendement de chlorure de trifluoroacétyle est de 93,2%.

*Exemple 5:*

En opérant à 188°C dans le même appareillage et sur le même catalyseur que dans l'exemple 3, la conversion du fluoral introduit dans le réacteur à raison de 4,12 Nl/h avec, pour 1 mol de fluoral, 1,43 mol de chlore et 4,76 mol d'acide chlorhydrique, est de 99% et le rendement de chlorure de trifluoroacétyle de 88,3%.

*Exemple 6:*

Dans l'appareillage de l'exemple 3, et en opérant à 220°C avec 0,3 l du catalyseur utilisé dans ce même exemple, la conversion du fluoral introduit dans le réacteur à raison de 4,26 Nl/h avec, pour 1 mol de fluoral, 1,32 mol de chlore, 1 mol d'acide chlorhydrique et 3,68 mol d'azote, est totale et le rendement en chlorure de trifluoroacétyle est de 94,2%.

*Exemple 7:*

En opérant à 210°C sur le même catalyseur et dans le même appareillage que dans l'exemple 3, le fluoral, introduit dans le réacteur à raison de 0,203 mol/h avec, pour 1 mol de fluoral, 1,4 mol de

gekennzeichnet, dass die Temperatur zwischen 130°C und 250°C liegt.

chlore, 1 mol d'acide chlorhydrique et 3,3 mol de chlorotrifluorométhane, est totalement transformé et le rendement de chlorure de trifluoroacétyle est de 96,9%.

## Revendications

1. Procédé de préparation de chlorure de trifluoroacétyle, caractérisé en ce que le trifluoroacétaldéhyde (fluoral) est chloré catalytiquement par le chlore en phase vapeur sur un charbon actif.

2. Procédé selon la revendication 1, caractérisé en ce que le rapport molaire chlore/fluoral est de 1 à 2.

3. Procédé selon l'une quelconque des revendications 1 et 2, caractérisé en ce que la température est comprise entre 130 et 250°C.

4. Procédé selon l'une quelconque des revendications 1 à 3, caractérisé en ce que la chloration est effectuée en présence d'une quantité d'acide chlorhydrique telle que le rapport molaire acide chlorhydrique/fluoral ne soit pas supérieur à 5.

5. Procédé selon l'une quelconque des revendications 1 à 3, caractérisé en ce que la chloration est effectuée en présence d'une quantité d'un diluant inerte telle que le rapport molaire diluant inerte/fluoral ne soit pas supérieur à 5.

6. Procédé selon l'une quelconque des revendications 1 à 3, caractérisé en ce que la chloration est effectuée en présence d'une quantité d'acide chlorhydrique et d'une quantité de diluant inerte telles que la somme des rapports molaires acide chlorhydrique/fluoral et diluant inerte/fluoral ne soit pas supérieure à 5.

7. Procédé selon l'une quelconque des revendications 5 et 6, caractérisé en ce que l'azote est choisi comme diluant inerte.

8. Procédé selon l'une quelconque des revendications 5 et 6, caractérisé en ce que le chlorotrifluorométhane est choisi comme diluant inerte.

9. Procédé selon l'une quelconque des revendications 1 à 8, caractérisé en ce que la chloration est effectuée en lit fixe.

10. Procédé selon la revendication 9, caractérisé en ce que le temps de contact est de 5 à 30 s.

11. Procédé selon l'une quelconque des revendications 1 à 8, caractérisé en ce que la chloration est effectuée en lit fluide.

12. Procédé selon la revendication 11, caractérisé en ce que la vitesse du mélange gazeux des réactifs est telle qu'elle corresponde à un débit 1,5 à 3 fois plus élevé que le débit minimum de fluidisation du catalyseur.

## Patentansprüche

1. Verfahren zur Herstellung von Trifluoracetylchlorid, dadurch gekennzeichnet, dass Trifluoracetaldehyd (Fluoral) mit Chlor in Gasphase mit einer Aktivkohle katalytisch chloriert wird.

2. Verfahren nach Anspruch 1, dadurch gekennzeichnet, dass das Chlor/Fluoral-Molverhältnis 1 bis 2 beträgt.

3. Verfahren nach Anspruch 1 oder 2, dadurch

4. Verfahren nach einem der Ansprüche 1 bis 3, dadurch gekennzeichnet, dass die Chlorierung in Anwesenheit einer solchen Menge Chlorwasserstoff erfolgt, dass das Molverhältnis Chlorwasserstoff/Fluoral nicht über 5 liegt.

5. Verfahren nach einem der Ansprüche 1 bis 3, dadurch gekennzeichnet, dass die Chlorierung in Anwesenheit einer solchen Menge an inertem Verdünnungsmittel erfolgt, dass das Molverhältnis inertes Verdünnungsmittel/Fluoral nicht über 5 liegt.

6. Verfahren nach einem der Ansprüche 1 bis 3, dadurch gekennzeichnet, dass die Chlorierung in Anwesenheit solcher Mengen Chlorwasserstoff und inertem Verdünnungsmittel durchgeführt wird, dass die Summe der Molverhältnisse Chlorwasserstoff/Fluoral und inertes Verdünnungsmittel/Fluoral nicht über 5 liegt.

7. Verfahren nach Anspruch 5 oder 6, dadurch gekennzeichnet, dass Stickstoff als inertes Verdünnungsmittel gewählt wird.

8. Verfahren nach Anspruch 5 oder 6, dadurch gekennzeichnet, dass das Chlortrifluormethan als inertes Verdünnungsmittel gewählt wird.

9. Verfahren nach einem der Ansprüche 1 bis 8, dadurch gekennzeichnet, dass die Chlorierung im Festbett erfolgt.

10. Verfahren nach Anspruch 9, dadurch gekennzeichnet, dass die Kontaktzeit 5 bis 30 Sekunden dauert.

11. Verfahren nach einem der Ansprüche 1 bis 8, dadurch gekennzeichnet, dass die Chlorierung im Fliessbett erfolgt.

12. Verfahren nach Anspruch 11, dadurch gekennzeichnet, dass die Geschwindigkeit der Gasmischung aus den Reaktionspartnern derart ist, dass sie dem 1,5- bis 3,0-fachen Durchsatz des Mindestdurchsatzes für die Fluidisierung des Katalysators entspricht.

## Claims

1. Process for the preparation of trifluoroacetyl chloride, characterised in that trifluoroacetaldehyde (fluoral) is catalytically chlorinated with chlorine in the vapour phase on an active charcoal.

2. Process as claimed in claim 1, characterised in that the molar ratio of chlorine to fluoral is from 1 to 2.

3. Process as claimed in claim 1 or 2, characterised in that the temperature is between 130 and 250°C.

4. Process as claimed in any one of claims 1 to 3, characterised in that the chlorination is effected in the presence of a quantity of hydrochloric acid such that the molar ratio of hydrochloric acid to fluoral is not more than 5.

5. Process as claimed in any one of claims 1 to 3, characterised in that the chlorination is effected in the presence of a quantity of an inert diluent such that the molar ratio of inert diluent to fluoral is not more than 5.

6. Process as claimed in any one of claims 1 to 3, characterised in that the chlorination is effected in the presence of a quantity of hydrochloric acid and a quantity of inert diluent such that the sum of the molar ratios of hydrochloric acid to fluoral and inert diluent to fluoral is not more than 5.

7. Process as claimed in claim 5 or 6, characterised in that nitrogen is used as the inert diluent.

8. Process as claimed in claim 5 or 6, characterised in that chlorotrifluoromethane is used as the inert diluent.

9. Process as claimed in any one of claims 1 to 8, characterised in that the chlorination is carried out in a fixed bed.

10. Process as claimed in claim 9, characterised in that the contact time is from 5 to 30 seconds.

11. Process as claimed in any one of claims 1 to 8, characterised in that the chlorination is effected in a fluidised bed.

12. Process as claimed in claim 11, characterised in that the velocity of the gaseous mixture of reagents is such as to correspond to a flow rate 1.5 to 3.0 times higher than the minimum flow rate of fluidisation of the catalyst.